# EUROPEAN PATENT APPLICATION

(11) **EP 0 776 674 A1**
(43) Date of publication of application: **04.06.1997**
(21) Application number: 95116575.2
(22) Date of filing: 20.10.1995
(51) Int. Cl.: A61N 1/05

(54) **Temporary atrial defibrillation catheter**

(71) Applicant: Alt, Eckhard, Dr., D-85521 Ottobrunn (DE)
(72) Inventor: Alt, Eckhard, Dr., D-85521 Ottobrunn (DE)
(74) Representative: Haft, von Puttkamer, Berngruber, Czybulka

(57) **Abstract**

Atrial fibrillation disorders are treated by locating low impedance electrodes positioned on a single catheter body passing through the pulmonary artery valve to locate the electrodes respectively in the right atrium and the pulmonary artery. Thus an electrical energy impulse of the order of three joules is passed through a heart and tissue path establishing a field gradient for resetting atrially fibrillating cells to convert atrial fibrillation into sinus rhythm. A special non-implantable catheter for temporary use provided for this treatment method is characterized by an inflatable balloon on the distal tip end, two low impedance energy discharging electrodes spaced to reside respectively in the right atrium and the pulmonary artery and a flexible polymer catheter body sheath of a diameter of the order of two millimeters. Inflating and deflating of the distal balloon facilitates the positioning of the catheter and guarantees a stable position during the application of the electrical energy shock for conversion of atrial fibrillation.

## Description

### TECHNICAL FIELD:

This invention relates to a catheter for the treatment of atrial fibrillation, as distinguished from ventricular fibrillation, and especially to an atrial catheter for discharge of electrical energy in the region of the heart to convert atrial fibrillation into sinus rhythm, and more particularly it relates to temporary monitoring and treatment of atrial arrhythmic disorders with non-implantable catheters threaded into the heart.

### BACKGROUND ART:

With current trends toward longer life expectancy the occurrence of atrial fibrillation increases significantly in all industrial nations. Currently atrial fibrillation is the main cause for hospitalization in the U. S. A. for arrhythmic disorders. It is also the most underlying reason for cerebrovascular stroke events. A solution to atrial fibrillation diseases are favored to correct its complication upon life and increase life expectancy, and also hemodynamics and thromboembolic complications favor a solution to such diseases by converting atrial fibrillation into sinus rhythm. However the use of antiarrhythmic drugs effective to preserve sinus rhythm produce a significant risk of proarrhythmias and reduced survival rate.

Also external shock with energies between 100 and 350 joules is a standard therapy for converting atrial fibrillation into sinus rhythm. The risks and complications of this type of therapy include late ventricular fibrillation, pericarditis due to the high electrical current and fractures of the spine and ribs following muscular contractions with high energy shock application.

The art of implanted atrial defibrillation electrodes using lower energies is well developed, as disclosed for example by J. Adams, et al. in U. S. Patent 5,282,837, Feb. 1, 1994 for Atrial Defibrillator and Method. Therein, defibrillating energy is released from an implanted defibrillator for discharge between two electrodes of an implanted catheter threaded through the coronary sinus with energy discharge electrodes respectively located in positions beneath the left atrium near the left ventricle and in a region adjacent the right atrium coronary sinus ostium for minimizing ventricular fibrillation potential. The implanted defibrillator senses arrythmia and controls the energy discharge.

Ventricular fibrillation is also arrested with implanted systems as disclosed by T. Shulte, et al. in U. S. Patent 5,269, 319 by means of two defibrillation electrodes on a single catheter inserted via the superior vena cava into the right atrium and right ventricular cavity respectively. This system discloses the manner in which the defibrillation impulse energy is synchronized with the R-waves and provides for detection of the R-waves for that purpose.

It is a general object of this invention to provide improved treatment of atrial fibrillation on a temporary basis and correct the foregoing deficiencies of the prior art.

A further objective of this invention is to significantly reduce the amount of energy required for treatment of atrial fibrillation from external impulse generators with a hybrid system of internal defibrillating electrodes powered by external energy and control means.

However there are problems related to prior art electrodes both in the necessity to implant and the requirement to process enough energy for defibrillation in circumstances. In particular the effort that it takes to install and operate implanted systems is not tolerable when life atrial fibrillation occurs only rarely. The nature of effective prior art electrodes is generally intrusive with large electrode areas necessary to handle the high energy impulses that can initiate rejection reactions in the human body. Also implantation of netting, barbs and anchors in the region of the heart is not tolerable and not feasible for only temporary systems.

There are such conditions as congestive heart failure which after defibrillation can require continuous monitoring rather than isolated shock treatment. Furthermore cardiac surgical conditions that need post-operative monitoring for atrial dysrhythmias and defibrillation, require instantaneous treatment when atrial fibrillation is detected.

Accordingly it is a further object of this invention to provide a method and accompanying instrumentation for temporary use for limited periods of time to continuously monitor and instantaneously to treat dysrhythmias and atrial fibrillation with adequate electric field gradient shock energy, without implantation.

Other objects, features and advantages of the invention will be found throughout the following description, the accompanying drawings and the claims.

### DISCLOSURE OF THE INVENTION:

The invention is characterized by the features of claim 1.

This invention answers the need for temporary but on line monitoring and treatment of atrial arrythmia by defibrillation. Such methods of treatment and associated improved catheter equipment can be used to reduce risks of complications from conventional shock conversion of atrial fibrillation into sinus rhythm. Thus dysrhythmias, congestive heart failure and postoperative problems after cardiac surgery can be treated with little delay and reduced risk incurred than with conventional methods.

In the method of treating atrial fibrillation disorders in accordance with this invention two low impedance electrodes positioned on a single catheter body are passed through the right atrium and pulmonary artery valve with one resident in the right atrium and the other resident in the pulmonary artery. This permits the discharge of electrical impulse energy of the order of three to seven joules from the electrodes to pass through the intervening heart blood and tissue path to establish a field gradient of sufficient amplitude for resetting atrially fibrillating cells and thereby converting atrial fibrillation into sinus rhythm. The patient is monitored continuously during high risk periods of time by external control and impulse generating equipment coupled to the internal catheter for instantaneous use under prescribed conditions.

This procedure is accomplished with a non-implantable atrial defibrillation catheter specially constructed for threading through the right ventricle and the pulmonary valve of the heart into the pulmonary artery to dispose temporarily two low impedance electrical impulse energy discharge electrodes respectively in the pulmonary artery and right atrium. The electrodes thereby encompass an area that permits reset of virtually all atrially fibrillating cells in the presence of a shock impulse that creates an adequate strength electric field gradient.

A distal tip end of said catheter carries an inflatable balloon connected by a lumen to a proximal end of the catheter for external control of inflation. This balloon serves two unique purposes, namely (1) when partly inflated it is a propellant agent acting as a vehicle with a sail for carrying the catheter tip with the flow of blood through the right ventricle and the pulmonary artery valve into the pulmonary artery, and ( 2 ) when fully inflated it serves as a temporary anchor in the pulmonary artery so that the portion of the catheter towards the proximal end that will reside in the heart and atrium can be shaped for its temporary resident position by forces exerted from the proximal end external to the body. When installed the balloon may be deflated to produce only nominal impediment to flow of blood in the pulmonary artery.

On the catheter, one of two low impedance energy dispersing defibrillation electrodes is disposed near the distal tip end of the catheter. The other defibrillation electrode is separated by a length of electrically insulating catheter sheath material of the order of twenty centimeters in length. Thereby the proximal defibrillation electrode is lodged in the right atrium when the distal defibrillation electrode is lodged in the pulmonary artery. In this position, the intervening electrically insulating catheter sheath passes through the pulmonary artery valve and lodges therein. To avoid interference with the functioning of the artery valve the catheter structure along the length of the insulating material separating the two defibrillation electrodes constitutes a flexible polymer sheath of a diameter in the order of two millimeters encompassing preferably a single flexible electrical lead connected from the distal defibrillation electrode to lead from the proximal end of the catheter. Also encompassed is an internal lumen leading to the distal end balloon for taking blood samples, for injection of fluids and for monitoring of the pulmonary artery pressure and of the pulmonary artery wedge pressure. This catheter structure permits the pulmonary artery valve to function for reasonable periods of time substantially normally in the presence of the catheter. An additional third ring electrode on the catheter situated in a position to detect the intracardiac ECG serves as sensing and pacing electrode for triggering of the defibrillation shock wave for pacing the heart.

Other objects, features and advantages of the invention are set forth throughout the following description, claims and the appended drawings.

### BRIEF DESCRIPTION OF THE DRAWINGS:

In the accompanying drawings, wherein like reference characters are used to facilitate comparison of similar features in the various views,
Figure 1 is a schematic sketch of the atrial defibrillation catheter lodged in place within the heart for defibrillation in accordance with the method of this invention,
Figure 2 is a diagrammatic, foreshortened sketch of the distal end region of the atrial defibrillation catheter structure afforded by this invention,
Figure 3 is a foreshortened sketch dimensionally setting forth detailed structure of one preferred embodiment of the atrial defibrillation catheter,
Figure 4 is a modified catheter with a lumen for continuous monitoring and infusion of fluids into the body, such as required in post-operative procedure, and
Figure 5 is a block sketch of the external control center for defibrillation in accordance with the method introduced by this invention.

### THE PREFERRED EMBODIMENT:

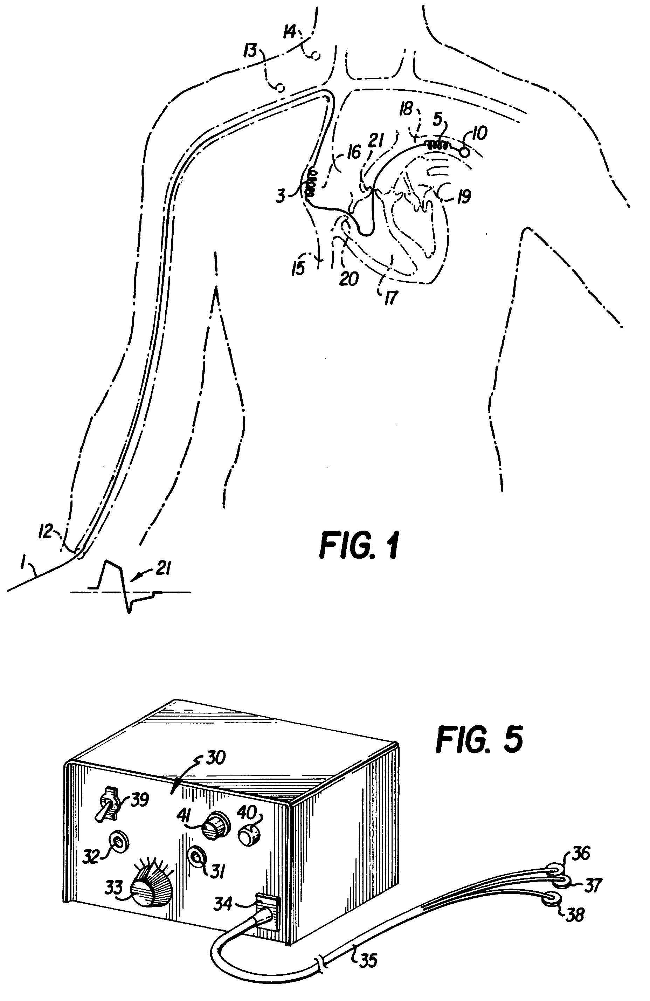

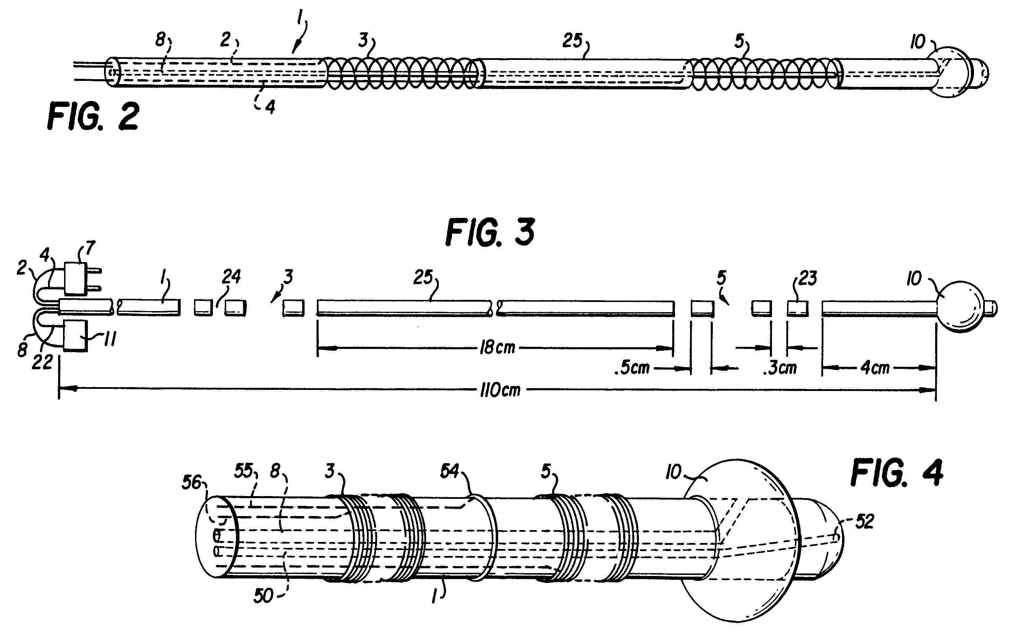

The sketch of the human body in Figure 1 shows a single catheter 1, inserted at the preferable brachial vein puncture site 12 in the right arm to pass through the right atrium 16 into the heart. Internally embedded conductors 2 and 4, better seen in Figures 2 and 3, terminate at the proximal end in an external electrical connector 7 which in turn provides electrical energy from the external atrial defibrillator energy source and control system 30 (Figure 5). These conductors 2 and 4 terminate at the distal end in the defibrillation electrodes 3 and 5 which are lodged respectively in the right atrium 16 and the pulmonary artery 18.

The catheter is constructed of a polymer sheath flexible enough to pass through the venous system of the human body and to attain the configuration shown in the heart. A typical diameter is two millimeters. The electrical conductors 2, 4 are flexible stranded wires for carrying enough impulse defibrillation energy to the respective defibrillation electrodes 3, 5 for defibrillation, typically in the order of about three to seven joules in accordance with this invention.

The structure of electrodes 3 and 5 is critical for defibrillation and thus requires a significant electrode surface exposure area to heart blood and tissue for transmitting the necessary shock impulse energy, preferably the shape of biphasic impulse 2 l. To produce the required surface area and to locate the electrodes in the respective atrium and pulmonary artery locations necessary for establishing the electric field path through the heart for atrial defibrillation, the electrodes are preferably constructed in the manner and with dimensions such as shown in Figure 3.

The overall length of catheter 1 therefore is 110 cm from distal end to proximal end, and the outside diameter is about two mm. The inflatable balloon 10 resides at the distal end tip and is inflated by way of an encompassed lumen 8 which extends from the catheter proximal end to a plug 11 that provides air control for inflation and deflation of the balloon, etc. by connection to an external syringe of 2 cc. Also for monitoring, for taking blood samples and infusion of body fluids, particularly critical at the post-operative stage, a further lumen 22 is provided to terminate at or near the distal end. This lumen has a dimension adapted to introduce a thin guide wire for easier application and positioning of the catheter. A suitable guide wire has for example a diameter of 0,65 millimeter so that this lumen 22 has a diameter with slightly larger dimensions.

Each of the defibrillation electrodes 3, 5 is constructed of a series of 0.5 cm long rings 23, typically nine, separated by 0.2 cm spacings 24. The distal electrode 5 is spaced about four cm from the balloon, and the two electrodes 3, 5 have an insulating sheath spacer 25 eighteen cm. long between them. The stainless steel rings 23 and interim spacings 24, which constitute insulating catheter sheath polymer material provide a smooth surface which does not irritate human tissue when passed through the venous system. The area and length of the rings therefore is sufficient to create an electric field gradient of adequate strength through the heart to reset substantially all atrially fibrillating cells and establish sinus rhythm with short duration shock impulse energy of an average of three joules, as confirmed by a study conducted by the applicant in patients with atrial fibrillation following an enlarged and diseased atrium.

The very flexible spacer sheath 25 between the electrodes requires only one electrical wire and is smooth and small diameter so that its position through the pulmonary artery valve 21 for reasonable periods of time will not adversely affect its functioning. Nor will this spacer affect the function of the tricuspid valve 20. Thus the catheter may be used in place for several days in a post-operative stage for monitoring and defibrillating in the event an atrial fibrillation is detected. Furthermore the flexible sheath polymer spacers 24 between the stainless steel rings 23 afford enough flexibility at the electrode sites to bend the catheter about body cavities during insertion and positioning in the heart. Other types of electrodes such as braided metal mesh serves as well for the purpose of shocking electrodes 3 and 5.

In the insertion of the catheter, the balloon 10 is inflated partly in order to facilitate the positioning of the defibrillation catheter 1 into the pulmonary artery 18. The inflation of the balloon in the right atrium 16 will help advance the catheter with the blood flow into the pulmonary artery 18. The inflated balloon 10 also helps maintain a stable position of the distal end of the catheter 1 in the pulmonary artery, for example, temporarily as the catheter 1 is fed into the heart to attain the position shown passing from the right ventricle 17 into the left pulmonary artery 18. The fixation of the distal tip serves additionally the aim of advancing the catheter 1 to manipulate the shocking electrode 3 into contact with the atrium 16 for good electrical contact when the defibrillation pulse is applied without dislodging the electrode 5 from its position in the pulmonary artery. Furthermore, inflation and deflation of the balloon 10 enables measurements of pulmonary wedge pressures.

The catheter 1 may alternatively be inserted at the subclavian puncture site 13 or the internal jugular site 14. Also, it would be possible to approach through the femoral vein and the inferior vena cava 15. In any event the catheter passes through the right atrium 16 to lodge the defibrillator electrode 3 therein, and extends through the right ventricle 17 and the pulmonary artery valve 21 into left pulmonary artery 18 to lodge the defibrillator electrode 5 in the pulmonary artery 18. This critical positioning permits a vector of electric energy to pass through the left atrium.

The external atrial defibrillator 30 is shown in Figure 5. The defibrillator has an internal logic unit which monitors and evaluates the intracardiac signal and other parameters important for the function of the system. It provides an electric shock impulse, preferably biphasic (21) of variable duration, preferably four milliseconds, with a second pulse of opposite polarity of two and one half milliseconds. Also it provides variable energy, as indicated by knob 33 in a range between one and ten joules. Plugs 31 and 32 permit the anode and cathode leads 2, 4 of the catheter 1 to be connected. For triggering the defibrillation shock pulse synchronously with the R-wave, the ECG lead 35 with surface electrodes 36, 37, 38 is provided. The external defibrillator 30 is turned on and off with switch 39. A temporary mode indicating correct synchronization with the R-wave is indicated by the LED 40 when the momentary switch 41 is depressed for testing without generating shock pulses. Other controls may include atrial fibrillation detectors that can be coupled to automatically generate a shock wave under prescribed conditions, and fluid controls for lumens 8 and 22 to monitor internal body fluids and provide for balloon inflation. Since for example the timing of the shock is critical for the success of conversion to sinus rhythm and for the energy requirements the information obtained from the additional ring electrode 54 can be used to analyse the ongoing atrial fibrillation with regards to phases of high and low success probability of conversion. There are some critical relations between fibrillation and conversion: The more organised fibrillation is present the higher is the low frequency content of the signal; the lower the high frequency component is in the signal the lower energy is required for conversion. Based on the analysis of these criteria an automatic shock timing function is incorporated in the external defibrillator. Also automatic safety features such as refractory periods that prevent shock application at rapid heart beats are incorporated in the defibrillator. In practice the timing of a shock delivery will be restricted to two successive heart beats occurring within a time period of 500 milliseconds. Moreover, the defibrillator may deliver two consecutive shocks within a period of 1 to 6 seconds, preferably 2 to 5 seconds, both triggered to the occurrence of an R-wave, in order to decrease the shock strengths of a single shock that may exceed the tolerable pain load of the patient. It has been shown that two shocks of tolerable pain level have the same or even better success than a single defibrillation shock of higher energy. In the application of two successive shocks in the said time period between 1 and 6 seconds the first shock acts as a preconditioning shock temporarely lifting the chaotic state of atrial fibrillation into a higher order of organisation; therefore, the second shock may only have a relatively low energy to bring the atrial fibrillation in the desired fully organized status. The monitoring and evaluating of the intracardiac signal includes the automatic R-wave-triggering and the automatic evaluation of the phases so that the consecutive shocks are applied with an optimum timing. The second shock is delivered with a substantially lower energy with respect to the first shock since the fibrillation is already in a higher order status after the application of the first shock.

Furthermore, the external defibrillator has a testing circuit to control the impedance and the correct position of the electrodes of the catheter. A test shock with very low energy which does not influence the heart rhythm is applied to the catheter. If the position of the electrodes and the catheter is correct then the impedance between the electrodes is less than approximately 60 Ohm. If the impedance is higher then the catheter must be repositioned. It has been shown in the practice that this value of impedance is a good criterion to control the correct position of the catheter.

The testing circuit as well as the logic circuit are connected to a display 61 for displaying functional parameters of the defibrillator, especially the intracardiac signal ECG and the trigger time during the testing phase. Therefore, a physician can monitor whether the triggering and the application of a shock take place only during the R-wave which is a characteristic of the cardiac depolarisation. Preferably the testing circuit has an adaptive filter circuit to filter the low frequency T-wave of the intracardiac signal.

In Figure 2, a foreshortened schematic sketch of the distal end of a catheter 1 provided in accordance with this invention shows the defibrillation electrodes 3, 5 separated by the intermediate separating catheter sheath section at the distal end. The balloon 10 is positioned at the distal end tip of the catheter 1. The internal lumen 8 operates the balloon, and may as shown in Figure 4 be accompanied by the additional lumen 50 that terminates at the distal end tip 52. The additional electrode ring 54 intermediate electrodes 3 and 5 together with conductor wire 55 is used for pacing and sensing of intrinsic intracardiac activity and together with electrodes 3 and 5 can be used to sense the R-wave in place of external ECG electrodes for correct triggering of the defibrillation shock to the R-wave in order to avoid the induction ventricular fibrillation following a non-synchronized shock application.

Accordingly this invention has advanced the state of the art by introducing a novel method of treatment of atrial disorders resulting in atrial fibrillation which incorporates novel electrode placements, non-implantable catheter instrumentation features and the ability to continuously monitor short term atrial behavior for such0 purposes as post-operative monitoring of cardiac surgery. Those features of novelty embodying the nature and spirit of the invention are defined with particularity in the following claims.

## Claims

1. A catheter for temporary insertion in the body of a patient to treat atrial fibrillation, comprising:
a thin elongate flexible catheter body having proximal and distal ends and electrical means including a pair of low impedance electrodes spaced apart on said catheter body and conforming thereto between the distal and proximal ends thereof, said electrodes being of sufficient size to establish an electric field through right and left atrial chambers of the patient's heart of sufficient strength to defibrillate the atria when the distal end of the catheter body is advanced to and positioned in the pulmonary artery of the patient's heart so that said electrodes are located to establish said electric field when energized by defibrillating electrical shock impulses, and further including electrical conductors connected to respective ones of said pair of electrodes and accessible at the proximal end of the catheter body for selective application of one or more debrillating electrical shock impulses thereto, and
deployable facilitating means at the distal end of the catheter body for selective deployment to aid maneuvering of the catheter body in advancement through the patient's heart and for temporarily and passively maintaining the distal end of the catheter body in the pulmonary artery to allow establishing said electric field through the right and left atrial chambers, and for redeployment to allow withdrawal of the catheter body.

2. The catheter of claim 1, wherein said electrical means further includes additional electrode means on the catheter body intermediate said spaced apart pair of electrodes, for pacing and sensing intracardiac activity of the patient's heart.

3. The catheter of claim 1, further including lumen means in the catheter body to allow taking blood samples from the patient when the distal end of the catheter body is disposed in the pulmonary artery.

4. The catheter of claim 1, further including lumen means running the length of the catheter body to enable measuring pressure in the pulmonary artery when the distal end of the catheter body is disposed therein.

5. The catheter of claim 1, wherein the spacing between said pair of electrodes relative to the distal and proximal ends of the catheter body is such that when the distal end of the catheter body is disposed within the pulmonary artery, the electrodes of said pair are positioned in the pulmonary artery adjacent the left atrium and in the right atrium of the patient's heart, respectively.

6. A catheter for temporary insertion in the body of a patient to treat atrial fibrillation, comprising:
a thin, flexible, elongate catheter body having a proximal end and a distal end, first and second spaced apart, relatively low impedance electrodes carried by the catheter body and confirming thereto, the first electrode being positioned at or near the distal end of the catheter body and the second electrode being positioned toward the proximal end thereof and separated from the first electrode by a distance which is predetermined to locate the second electrode in the right atrium of the patient's heart when the first electrode is located in the patient's pulmonary artery by having advanced the distal end of the catheter body through a path including the right atrial and ventricular chambers and into the pulmonary artery of the heart,
a balloon attached adjacent the distal end of the catheter body and coupled by a lumen in the catheter body to the proximal end thereof for selective inflation and deflation of the balloon, to facilitate advancing the distal end of the catheter body through a portion of a path through the patient's heart in the direction of blood flow when the balloon is partially inflated, until the first electrode is positioned in the pulmonary artery adjacent the left atrium of the patient's heart, whereby to position the second electrode in the rigth atrium by virtue of said spacing between the first and second electrodes, said balloon further serving to anchor the distal end of the catheter body in place in the pulmonary artery when the balloon is inflated sufficiently therein, to maintain the first and second electrodes positioned in the pulmonary artery and right atrium, respectively, and to permit the catheter body to be released and withdraw from the patient's heart when the balloon is deflated, and
first and second electrical conductor means carried by the catheter body from the proximal end thereof and connected to the first and second electrodes, respectively, for application of electrical energy pulses of sufficient magnitude thereto, and said first and second electrodes being of sufficient size, to establish an electrical field therebetween for atrial defibrillation of the patient's heart.

7. A catheter for temporary insertion in the body of a patient to treat atrial fibrillation of the patient's heart, comprising:
a flexible, elongate catheter body with distal and proximal ends and having a sufficient small diameter and sufficient flexibility to be threaded through a path inclucing right atrium, tricuspid valve, right ventricle, and pulmonary valve of the patient's heart, to position the distal end of the catheter body in the pulmonary artery;
first and second electrodes on said catheter body having respective first and second poles spaced apart for locating said first pole in the right atrium and said second pole in the pulmonary artery adjacent the left atrium of the patient's heart when the catheter body is threaded through said path into temporary position in the patient's heart with the distal end of the catheter body in the pulmonary artery, and electrical conductors connected to said first and second poles and traversing said catheter body to permit establishing an electric field between said poles when a defibrillating electrical shock waveform is applied to said conductors.

8. The catheter of claim 7, further including balloon means disposed at the distal end of said catheter body and coupled by a lumen to the proximal end of th catheter body to allow the balloon means to be partially inflated via said passage for steering the catheter body through a portion of said path under influence of blood flow along said path to position said first and second poles of said electrodes in location in the right atrium and pulmonary artery, respectively, and to be fully inflated for retention of the catheter body with said poles in said position, and to be deflated for withdrawal of said catheter body from the patient's heart after delivery of one or more defibrillating shocks waveforms through the atria via said poles.

9. A non-implantable atrial defibrillation catheter for threading through the pulmonary valve of the heart into the pulmonary artery to dispose temporarily two lwo impedance electrical impulses energy discharge electrodes respectively in the pulmonary artery and right atrium, comprising in combination,
one of said two electrodes being disposed near the distal tip end of the catheter in the pulmonary artery and the other separated by a length of electrically insulating material of the order to twenty centimeters to thereby lodge said other electrode inthe right atrium when the one said electrode is lodged in the pulmonary artery,with said electrically insulating material passing through the right heart and pulmonary artery valve, wherein the catheter structure along the length of the insulating material constitutes a flexible polymer sheath of a diameter in the order of two millimeters encompassing a flexible electrical lead from the one said electode disposed near the distal end of the catheter and said lumen thereby providing a structure that permits the pulmonary artery valve to function substantialy normally inthe presence of the catheter.

10. The catheter of claim 9 further comprising an inflatable balloon disposed at the distal tip end of the catheter connected by a lumen to a proximal end of the catheter for control of inflation thereof.

11. An external defibrillator connected to a catheter, preferably according to one of the preceeding claims, and providing an external shock impulse, preferably a biphasic shock synchronously with the R-wave of the intracardiac signal, characterized in that a logic and evaluation means is provided monitoring the low and high frequency contents of the intracardiac signal, said means determining thereof the organisation of the atrial fibrillation and controlling the energy of the external shock impulse in accordance to the determined organisation so that the provided energy is lower the higher the organisation of the fibrillation is.

12. The external defibrillator according to claim 11, characterized by testing means, said testing means delivering a testing shock impulse which does not influence the function of the heart, said testing means determining the impedance between the electrodes of the catheter and providing a warning signal if the impedance increases a predetermined value.

13. The defibrillator according to claims 11 or 12, characterized in that a display (61) is provided for displaying especially the intracardiac signal and the triggering time of the shock impulses.

14. The defibrillator according to one of the claims 11 to 13, characterized in that the defibrillator contains an adaptive filtering means for filtering the low frequency content of the T-wave in the intracardiac signal.

15. The defibrillator according to claim 11, characterized in that the logic means is provided with a delay circuit controlling the function of an impulse generator and transmitting control signals to the impulse generator so that the impulse generator delivers two consecutive shock impulses separated by a time period between 1 and 6 seconds, preferably 2 and 5 seconds, whereby the second impulse has a substantially lower energy content than the first impulse.
